# EUROPEAN PATENT SPECIFICATION

(11) **EP 2 263 637 B1**
(45) Date of publication and mention of the grant of the patent: **06.03.2013**
(21) Application number: 08739970.5
(22) Date of filing: 07.04.2008
(51) Int. Cl.: A61J 3/07, A61J 1/03, A61K 9/56

(54) **MEDICINAL PREPARATION FOR ORAL ADMINISTRATION**
MEDIZINISCHE ZUBEREITUNG ZUR ORALEN VERABREICHUNG
PRÉPARATION MÉDICINALE POUR ADMINISTRATION ORALE

(43) Date of publication of application: 22.12.2010
(73) Proprietor: Tsukioka Film Pharma Co., Ltd., Gifu, 509-0109 (JP)
(72) Inventor: TSUKIOKA, Tadao, Gifu 5008212 (JP); NISHIMURA, Misao, Gifu 5090123 (JP)
(74) Representative: Müller Schupfner & Partner
(86) International application number: PCT/JP2008/056867
(87) International publication number: WO 2009/125464

(56) References cited:
- WO-A2-2004/089336
- JP-A- 10 234 820
- JP-A- 2007 070 344
- JP-T- 2002 524 204
- US-A- 2 836 291
- US-A1- 2007 088 576

## Description

### TECHNICAL FIELD

The present invention relates to a formulation for oral administration.

### PRIOR ART

Recently, persons with dysphagia tend to increase in association with acceleration of aging. Therefore, there has been a strong demand for development of formulations which can be orally administered with ease to patients who have difficulty in swallowing a drug. Under such circumstance, the present inventors have proceeded research and development and proposed a film formulation in which a thin edible film contains a drug component. A film formulation proposed by the inventors collapses or dissolves rapidly in the oral cavity, at the same time, has a satisfactory practical strength in handling. Therefore people with decreased swallowing function due to aging, disease or the like, and those with low swallowing function, such as children, can also easily take the formulation.

Formulations for oral administration made from edible films are known from e.g. WO-A-2004/089336 and US-A-2836 291. Problems to be solved by the Invention

However, it has been difficult to contain a large amount of drug in a thin film in the above film formulation. Therefore, the above film formulation has had a problem that patients have to take many film formulations per dose when a large amount of drug needs to be taken in order to obtain drug action, although the formulation is extremely effective when a drug of which drug action can be obtained with a small amount thereof is used.

Further, in a manufacturing step of a film formulation, a step of dissolving or suspending a drug component in a solvent containing a film forming agent is required to be experienced, and a heating step is also required in some case. Therefore, it has been difficult that the film formulation contains a component, such as lactic acidbacteria or enzyme, which possibly causes degeneration or deterioration by mixing the component with a solvent or heating. In addition, among drugs, there are drugs which react with a film forming agent and inhibit the formation of a film. For example, a drug containing calcium ion becomes gel which doesn't dissolve even when heated, in reaction with sodium alginate as the film forming agent so that it is difficult to form a film. Therefore, there has been a problem that kinds of drugs which can be contained in the above film formulation are limited.

In addition, the above film formulation which collapses or dissolves rapidly in the oral cavity is also required to be a formulation which can be easily taken by persons with dysphagia.

Then, the present invention has been made in consideration of the above circumstances, an object thereof is to provide a formulation for oral administration, which can be easily taken even by persons with dysphagia, which contain a large amount of drug and does not limit kinds of drugs contained.

### Means for solving the problems

In order to solve the above problems, a formulation for oral administration according to the present invention includes "a liquid storing portion in which peripheries of overlaid water-soluble edible films are bonded, a closed space is formed inside the films, water-insoluble layers are laminated inside the water-soluble edible films and an aqueous liquid is stored inside the layers, and a drug storing portion which is adjacent to the liquid storing portion and in which peripheries of overlaid edible films are bonded and a closed space is formed inside the films and a drug is stored inside the space".

A "water-soluble edible film" constituting the liquid storing portion may be formed with a water-soluble film forming agent. Examples of the water-soluble film forming agent include gelatin, pectin, arabinoxylan, soybean polysaccharides, sodium alginate, carrageenan, xanthan gum, guar gum, pullulan, hypromellose (HPMC: name by Japanese Pharmacopoeia "hydroxypropyl methyl cellulose"), hydroxypropylcellulose (HPC), water-soluble hydroxyethylcellulose (HEC), methylcellulose (MC), propylcellulose, carboxymethylcellulose (CMC), polyvinyl alcohol (PVA), polyvinylpyrrolidone (PVP), polyethyleneglycol polyethylene oxide (PEO), polyethyleneglycol (PEG) and the like. Further, a plurality of types of film forming agents can be appropriately combined for use.

An "edible film" constituting the drug storing portion may be formed with a water-soluble, gastric-soluble or enteric film forming agent. Here, as the water-soluble film forming agent, the above-described agents can be used. And the agent may be same as or different from a film forming agent which forms the liquid storing portion. Also, as the gastric-soluble film forming agent, there can be exemplified by polyvinyl acetal diethylaminoacetate, polyvinyl aminoacetal, methyl methacrylate, butyl methacrylate, dimethylaminoethyl methacrylate copolymer, aminoalkyl methacrylate copolymer, and the like. Further, as the enteric film forming agent, there can be exemplified by hydroxypropyl methylcellulose acetate succinate (AQOAT), hydroxypropyl methylcellulose phthalate (HP), hydroxymethylethyl cellulose phthalate, carboxymethylethyl cellulose (CMEC), cellulose acetate trimeritate, cellulose acetate phthalate, cellulose acetate succinate, methyl cellulose phthalate, ethylhydroxyethyl cellulose phthalate, methacrylate copolymer L, methacrylate copolymer LD, ethyl ester methacrylate/acrylate copolymer, methyl ester methacrylate/methacrylate copolymer, styrene acrylate copolymer, methylmethacrylate/methacrylate copolymer, ethyl methacrylate/acrylate copolymer and the like.

It is to be noted that the water-soluble edible film constituting the liquid storing portion and the edible film constituting the drug storing portion may contain other components in addition to the above film forming agent. For example, flexibility of the edible film can be enhanced and occurrence of a crack or a break can be prevented by containing, as a plasticizer, triethyl citrate, glycerin, sorbitol, triacetin, propylene glycol, polyoxyethylene (105) polyoxypropylene (5) glycol, polysorbate, macrogol, glyceryl monostearate, mannitol or the like. The film can be seasoned or flavored by containing a sweetening agent such as sucralose, aspartame, saccharine and stevia, a taste corrigent such as xylitol and glycyrrhiza, or a flavoring such as spearmint flavor, lemon flavor and mentol. Alternatively, degeneration or deterioration of the film itself or content stored inside the film due to light or heat can be suppressed by coloring the film with a coloring agent. Further, coloring of the film can be also utilized for identifying the kind of drug stored in the drug storing portion.

A "water-insoluble layer" may be formed with, for example, a water-soluble polymer which has been crosslinked with a metal salt, a water-insoluble polymer or a hydrophobic material. Here, as the water-insoluble polymer, there can be exemplified by ethyl cellulose, propyl cellulose, acetyl cellulose, cellulose acetate propionate, hydroxypropyl cellulose phthalate, and cellulose acetate phthalate. Also, as the hydrophobic material, there can be used natural resin such as shellac, bees wax, sugar cane wax, carnauba wax, rosin, sandarac and zein, petrolatum, paraffin or silicone resin. Meanwhile, "lamination" of the water-insoluble layer can be performed by application with a coater machine, spraying or printings such as gravure printing and serigraph.

Herein, a "water-insoluble layer" is a layer for providing water-insolubility to the water-soluble edible film such that the water-soluble edible film doesn't dissolve with an aqueous liquid. However, in the case where the water-soluble edible film dissolves and the water-insoluble layer is a thin layer not having the strength to the extent that an internal space storing the aqueous liquid can be held, if the water-soluble edible film dissolves with saliva, the liquid storing portion collapses naturally. On the other hand, in the case where the water-insoluble layer has a certain level of strength, by applying teeth to the liquid storingportion, the water-insoluble layer which holds the internal space storing the aqueous liquid collapses in a ruptured manner so that the liquid storing portion collapses in accompanied with dissolution of the water-soluble edible film.

As a form where a water-soluble edible film constituting the liquid storing portion or an edible film constituting the drug storing portion is "overlaid", a form where two films are overlaid and a form where a film is folded and overlaid can be exemplified.

"Bonding" of peripheries can be performed by heat-sealing with external heating or heat-sealing with internal heating with radio waves or ultrasonic waves. Alternatively, bonding can be performed by non-heat-sealing using, for example, starch which is gelatinized and has high viscosity as an additive. Meantime, by using concave and convex shapes for sealing, embossing process can be performed. In addition, a plurality of bonding methods can be used in combination.

As an "aqueous liquid", there can be exemplified by water, aqueous solution, suspension through the medium of water and emulsion through the medium of water. More specially, there can be exemplified by water, sugar solution, saline solution, tea, fruit juice, gelatin solution, milk and yoghurt drink. Further, the aqueous liquid may be seasoned or flavored with a taste corrigent or a flavoring agent.

Forms of "drugs" are not particularly limited and examples thereof include powder (dispersant), granular, pill, tablet, liquid and paste-like formulations. Kinds of drugs are not particularly limited as long as the drug is for oral ingestion, and may be so-called a Western medicine or a Chinese medicine which is formed by a herbal medicine or herbal medicines in combination. Further a "drug" according to the invention includes dietary supplements which is not in the form of ordinary foods, such as granular and tablet, among foods for supplying scrimpy nutrients such as vitamin and mineral.

Here, shapes of the liquid storing portion and the drug storing portion are not particularly limited and may be polygonal shapes such as quadrangular, triangular, pentagon and hexagon, circular or oval in a planar view. Further when shapes thereof are polygonal shapes such as quadrangular, a user feels soft in his/her oral cavity, and can feel a sense of safety by making the angled portions round or making the contours waveform. Therefore it is preferable.

With the above structure, according to the present invention, the liquid storing portion and the drug storing portion can be taken simultaneously since they are adjacent to each other. Then, an aqueous liquid held by laminating the water-insoluble layer inside in the liquid storing portion spreads in the oral cavity since the water-soluble edible film dissolves with saliva and the liquid storing portion collapses. Therefore, when the edible film constituting the drug storing portion is water-soluble, the drug storing portion easily dissolves with aid of the aqueous liquid, a drug stored in the drug storing portion can be easily swallowed with aid of the aqueous liquid. Further, even when the edible film constituting the drug storing portion is water-insoluble, the drug storing portion can be easily swallowed with aid of the aqueous liquid.

In addition, when the drug storing portion is formed with the water-soluble edible film, the drug storingportion dissolves or collapses with saliva and the edible filmbecomes jelly-state. As a result, a drug can easily pass through the throat, since the jelly-state film wraps the drug or is intertwined with the drug. This enables the drug to be taken with the possibility of choking reduced even though the drug is fine powder or granular. Further, people can take the drug without feeling strong bitter taste or stimulation thereof because the drug is wrapped with the jelly-state film even for the drug with strong bitter taste, smell and stimulation. In addition to the action by such water-soluble edible film, the aqueous liquid is supplied from the liquid storing portion as described above so that the drug can be easily swallowed. Further, surface roughness of the drug can be reduced with the aqueous liquid.

On the other hand, when the water-soluble drug storing portion dissolves or collapses with saliva, the drug can be easily absorbed through the mucosa in the oral cavity so that the drug action appears rapidly compared with the gastrointestinal absorption. Therefore, it is suitable for the drug required to be fast acted, however, among drugs, there are drugs of which drug components are suitable to be acted gradually on the human body. Accordingly, when such drug is stored, the drug can be made gastric-soluble or enteric by forming the drug storing portion with gastric-soluble or enteric edible film, even though the drug itself has not been processed to adjust the solubility thereof.

Since the drug storing portion formed with gastric-soluble or enteric edible film doesn't dissolve or collapse in the oral cavity, people swallow the whole drug storing portion containing a drug. Therefore it cannot be easily swallowed without a fluid such as water. Compared to this, in the present invention, by simultaneously taking the liquid storing portion adjacent to the drug storing portion, people can easily swallow the drug storing portion without separately preparing water or the like because the fluid can be supplied from the liquid storing portion. Also, bitter taste, smell and stimulation of the drug can be masked with the drug storing portion which doesn't dissolve or collapse in the oral cavity. Further, the drug storing portion can be formed thinner with the edible film and doesn't take much space in the oral cavity so that it can be easily taken compared with the case where the drug is taken by wrapping the drug with an oblate and folding it or capsules.

In addition, a formulation for oral administration according to the present invention can be readily taken at any time and any place as water or the like is not required to be separately prepared. That is, the formulation for oral administration according to the present invention is excellent in portability.

Further, when the aqueous liquid has an original taste or flavor, such as sugar solution or fruit juice, or when the aqueous liquid has been seasoned or flavored, drug-derived bitter taste, smell or stimulation sensed when the drug storing portion dissolves in the oral cavity can be effectively masked with the aqueous liquid. And the aqueous liquid is seasoned to obtain a formulation for oral administration, which can be easily taken even by children.

Further, as the drug storing portion is formed into a sac-like form with the edible film, a large amount of drug can be stored compared to a case where the film itself carries a drug. Even a drug inhibiting the formation of a film can be stored in the drug storing portion. Alternatively, unlike the case where the film itself contains a drug, since the drug doesn' t need to be dissolved/suspended in the solvent or heated, even a drug which possibly degenerates/deteriorates by mixing the drug with a solvent or heating can be stored in the drug storing portion. That is to say, a formulation for oral administration can be produced without limiting kinds of drugs which can be applied.

Also, when the film itself contains a drug, if the film is tried to be cut into rounded shape comfortable in the oral cavity, disposal portions contain the drug so that drugs are wasted with unfavorable effect on environment. With respect thereto, in the present invention, a sufficient amount of drug can be stored in the sac-like drug storing portion so that the drug doesn't have to be contained in the edible film forming the drug storing portion. For this reason, even when edges of the edible film are cut such that the film is tried to be cut in a rounded shape, the disposal portions don't contain the drug. Therefore the drug can be effectively utilized with favorable effect on environment. Further, since the edible film doesn't contain the drug, the edible film is not affected by the hygroscopic property of the drug or the like in addition that the film has higher film strength than the case where the film contains the drug component. As a result, the nature of the drug storing portion can be stabilized.

The formulation for oral administration according to the present invention can be a formulation in which "the edible films constituting the drug storing portion are formed with the same film forming agent as the water-soluble edible films constituting the liquid storing portion and the edible films constituting the drug storing portion are laminated with the gastric-soluble or enteric layer."

The "gastric-soluble layer" can be formed by using the above gastric-soluble film forming agent, the "enteric layer" can be formed by using the above enteric film forming agent. Also, "lamination" of the gastric-soluble or enteric layer can be performed by application with a coater machine, spraying or printings such as gravure printing, serigraph. Meanwhile, the "gastric-soluble or enteric layer" may be formed outside or inside the edible film constituting the drug storing portion.

With the above structure, according to the present invention, the gastric-soluble or enteric drug storing portion and the water-soluble liquid storing portion can be formed with a same film as a substrate. This makes production steps of a formulation for oral administration simple. For example, in awater-soluble edible film, a water-insoluble layer is laminated on a portion corresponding to the liquid storing portion and a gastric-soluble or enteric layer is laminated on a portion corresponding to the drug storing portion. Such films are overlaid and bonded to each other so that the liquid storing portion and the drug storing portion can be formed simultaneously.

Meanwhile, the gastric-soluble or enteric layer can be a porous structure in which many spaces are formed. With this, the aqueous liquid or water in the oral cavity is absorbed to the spaces and the gastric-soluble or enteric layer increases in flexibility so that the drug storing portion can easily pass through the throat.

The formulation for oral administration according to the present invention can be the formulation "constituted by one liquid storing portion and two drug storing portions."

With the above structure, according to the present invention, drugs respectively stored in two drug storing portions can be taken simultaneously with the aqueous liquid stored in one liquid storing portion. It is to be noted that drugs different in components and dosage forms from each other can be stored in two drug storing portions. With this, a plurality of kinds of drugs can be taken at once without any troubles.

Alternatively, two drug storing portions may have different solubility from each other, for example, one drug storing portion may be water-soluble while the other drug storing portion is enteric. Therefore, for example, even when same kind of drug is stored in two drug storing portions, the drug stored in the water-soluble drug storing portion is absorbed through the mucosa in the oral cavity and provides drug action rapidly while the drug stored in the other drug storing portion is absorbed through the stomach or intestine and can be adjusted to provide drug action late. That is, even when the drugs themselves are not processed to adjust solubility thereof, times until the drug actions appear can be different from one another by adjusting the solubility of the drug storing portions so that drugs stored in two drug storing portions can be simultaneously taken.

The formulation for oral administration according to the present invention can be the formulation in which "each of the liquid storing portion and the drug storing portion includes a not-bonded portion which is not bonded along at least a portion of the outer circumference of the peripheral bonded portion."

"At least a portion of the outer circumference of the bonded portion" may be, for example, an outer circumference of the drug storing portion excluding a portion adjacent to the liquid storing portion or an outer circumference of the liquid storing portion excluding a portion adjacent to the drug storing portion. In this case, only bonded portion is present at the boundary of the drug storing portion and the liquid storing portion. Alternatively, in each of the drug storing portion and the liquid storing portion, not-bonded portions may be formed along the whole outer circumference of the bonded portion. In this case, the not-bonded portion interposes between the drug storing portion and the liquid storing portion.

In the bondedportion in which the overlaid films are bonded, the films have a thickness for two films. Therefore, some people possibly feel discomfort in their oral cavities. With respect thereto, in the present invention, the not-bonded portions, which are portions in which the overlaid films are not bonded and flitter as they are, exist outside the bonded portions in each of the drug storing portion and the liquid storing portion. Therefore, the not-bonded portions dissolve and are softened easily with saliva, compared to the bonded portion. As a result, people feel the formulation for oral administration softly in their oral cavities and can take more easily. Further if a portion which flitters exists, since feeling when picked up and visual impression are made to be soft, a sense of anxiety or resistance that the films possibly damage the oral cavity can be eliminated and films can be easily taken even when the films have linear and angled shapes. Meanwhile, the not-bonded portions can be provided in the width of 0.5 mm to 5 mm.

Also, when a not-bonded portion exists between the drug storing portion and the liquid storing portion, a film can be easily folded at this portion. As a result, the drug storing portion and the liquid storing portion can be easily taken by folding the film so that a formulation for oral administration doesn't take much space in the oral cavity and can be easily taken.

### Effect of the Invention

As described above, as an effect of the present invention, there can be provided a formulation for oral administration, which can be easily taken by persons with dysphagia, which contain a large amount of drug and does not limit kinds of drugs contained.

### Brief Description of the Drawings

Fig. 1 is a planer view showing a structure of a formulation for oral administration which includes one liquid storingportion and one drug storing portion.
Fig. 2 is a cross-sectional view cut along the line X-X in Fig. 1.
Fig. 3 is a planer view illustrating a method of producing the formulation for oral administration in Fig. 1.
Fig. 4 is a planer view illustrating a method of producing the formulation for oral administration in Fig. 1.
Fig. 5 is a planer view showing a structure of a formulation for oral administration including one liquid storing portion and two drug storing portions.
Fig. 6 is a cross-sectional view cut along the line Y-Y in Fig. 5.
Fig. 7 is a planer view illustrating a method of producing the formulation for oral administration in Fig. 5.
Fig. 8 is a planer view illustrating a method of producing the formulation for oral administration in Fig. 5.
Fig. 9 is a planer view showing a structure of a formulation for oral administration according to another embodiment.
Fig. 10 is a planer view showing a structure of a formulation for oral administration according to another embodiment.
Fig. 11 is a planer view showing structures of a formulation for oral administration and a formulation sheet according to another embodiment.
Fig. 12 is a planer view showing structures of a formulation for oral administration and a formulation sheet according to another embodiment.
Fig. 13 is a planer view showing structures of a formulation for oral administration and a formulation sheet according to another embodiment.

### Best Mode for Carrying out the Invention

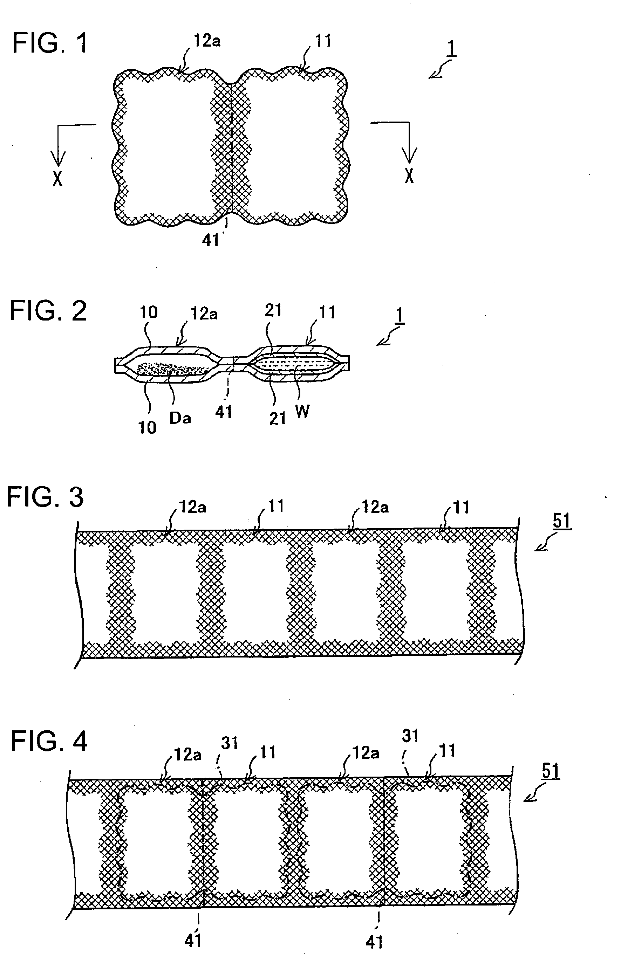

Hereinafter, a formulation for oral administration according to a preferable embodiment of the present invention will be described with reference to Fig. 1 to Fig. 4. In Fig. 2, the dimension in the direction of the thickness is enlarged for showing a structure apparently. Further, shaded portions in planar views show bonded portions in which edible films are bonded.

A formulation for oral administration 1 of the embodiment includes a liquid storing portion 11 in which peripheries of overlaid water-soluble edible films 10 are bonded and a closed space is formed inside the films and water-insoluble layers 21 are laminated inside the water-soluble edible films 10 and an aqueous liquid W is stored inside the layers, and a drug storing portion 12a which is adjacent to the liquid storing portion 11 and in which peripheries of overlaid edible films 10 are bonded and a closed space is formed inside the films and a drug Da is stored inside the space. Further, the drug storing portion 12a according to the embodiment is formed with a film forming agent which is the same kind of that of the water-soluble edible films 10 constituting the liquid storing portion 11.

As will be described in detail, the formulation for oral administration 1 of the embodiment includes one liquid storing portion 11 and one drug storing portion 12a. Also, the formulation for oral administration 1 is formed into a form in which the liquid storing portion 11 and the drug storing portion 12a, which have the approximately same shape and size, are continuously designed. The liquid storing portion 11 and the drug storing portion 12a are formed into substantially quadrangular shapes in a planar view (about 20 mm x about 30 mm) in which water-soluble edible films 10 each having a thickness of about 50 µm are overlaid and peripheries of the films are bonded. Then the formulation for oral administration 1 is formed into a waveform along the outer circumference. Here, the drug Da is stored in the drug storing portion 12a, a kind of aqueous liquid W among aqueous liquids exemplified by water, sugar solution, fruit juice, yoghurt drink and gelatin solution containing a sweetening agent is enclosed in the liquid storing portion 11. Meanwhile, 3 to 5 ml of the aqueous liquid can be stored in the liquid storing portion 11 according to the embodiment.

The formulation for oral administration 1 having such a structure can be produced as follows, for example. First, a solution of a water-soluble film forming agent is prepared. In this step, the water-soluble film forming agent is mixed with a liquid medium and the mixture is sufficiently stirred to dissolve the water-soluble film forming agent. At this time, the water-soluble film forming agent can be dissolved under heating. Also, additives such as plasticizer, flavoring agent or emulsifying agent can be added to the mixture. Here, as a liquid medium, water, hot water, dilute acidic solution, dilute alkaline solution or the like can be used, and organic solvents such as alcohol can be added depending on kinds of the water-soluble film forming agent.

Subsequently, a base film (for example, manufactured by PP, PET) is fixed to a smooth surface and the prepared solution of the water-soluble film forming agent is applied uniformly by flow-casting over the base film. Then flow-casted solution is dried to form the water-soluble edible film 10.

Then, the water-insoluble layer 21 is laminated on a portion of the water-soluble edible film 10 corresponding to the liquid storing portion 11. For example, the water-insoluble layer 21 can be laminated on the water-soluble edible film 10 by partially coating a solution of water-soluble polymer crosslinked or water-insoluble polymer with application, spraying, printing or the like. Alternatively, a hydrophobic material such as shellac and bees wax may be applied. Then when the water-insoluble layer 21 is dried, the base film is stripped from the water-soluble edible film 10.

Next, two water-soluble edible films 10 on which the water-insoluble layers 21 are partially laminated are overlaid on one another such that the water-insoluble layers 21 position inside the water-soluble edible films 10, and bonded to each other excluding a portion thereof so as to open the films. According to the embodiment, the liquid storing portion 11 and the drug storingportion 12a which are substantially quadrangular shapes are bonded along three sides respectively and opened along the remaining one side.

Then, the aqueous liquid W is injected from the opening of the liquid storing portion 11 and the drug storing portion 12a is filled with the drug Da from an opening thereof. Subsequently, when openings of the liquid storing portion 11 and the drug storing portion 12a are bonded and sealed, a formulation sheet 51 in which the liquid storing portions 11 and the drug storing portions 12a are continuously designed one after another is produced as shown in Fig. 3. Meanwhile, the base films may be stripped after the water-soluble edible films 10 including the base films are bonded and the liquid storing portion 11 and the drug storing portion 12a are formed.

Finally, as shown in Fig. 4, the formulation for oral administration 1 shown in Fig. 1 and Fig. 2 can be obtained by cutting the formulation sheet 51 along dashed-dotted lines 31 shown in the drawing in the bonded portions. Meanwhile, in the present embodiment, perforated lines 41 are provided between the liquid storing portions 11 and the drug storing portions 12a for easily folding the formulations for oral administration 1. Also, the obtained formulations for oral administration 1 can be packed individually with packs, each of which is formed with, for example, a metal foil such as aluminum foil or a transparent plastic film having high gas barrier property.

Meanwhile, although sealing is performed by bonding with external heating (heat sealing) in the present embodiment, layers having excellent heat sealing property may be previously laminated inside portions to be bonded of the water-soluble edible films 10. The layer having excellent heat sealing property can be formed by using, for example, gelatin, sodium caseinate, zein, polyvinyl alcohol, polyvinyl pyrrolidone, starch, processed starch, pullulan, arabinoxylan, or soy protein.

According to the formulation for oral administration 1 having such structure, the liquid storing portion 11 and the drug storing portion 12a can be simultaneously taken, and the liquid storing portion 11 and the drug storing portion 12a formed with the water-soluble edible films 10 dissolve or collapse with saliva at the same time. As a result, the drug Da stored in the drug storing portion 12a can be easily swallowed with aid of the aqueous liquid W stored in the liquid storing portion 11. Therefore, with the formulation for oral administration 1, the drug Da can be orally administered to a person with dysphagia. Further, the formulation for oral administration 1 can be easily taken and is excellent in portability since the formulation can be taken without preparing water and the like.

Further, as the drug storing portion 12a is formed into a sac-like form with the water-soluble edible films 10, a large amount of drug can be stored compared to a case where films themselves carry the drug. Even a drug inhibiting the formation of a film can be stored in the drug storing portion 12a. Alternatively, unlike the case where films themselves contain a drug, since the drug doesn't need to be dissolved/suspended in the solvent or heated, even a drug which possibly degenerates/deteriorates by mixing the drug with a solvent or heating can be stored in the drug storing portion 12a. That is to say, the drug storing portion 12a can be applied to a formulation for oral administration 1 according to the embodiment without limiting kinds of drugs.

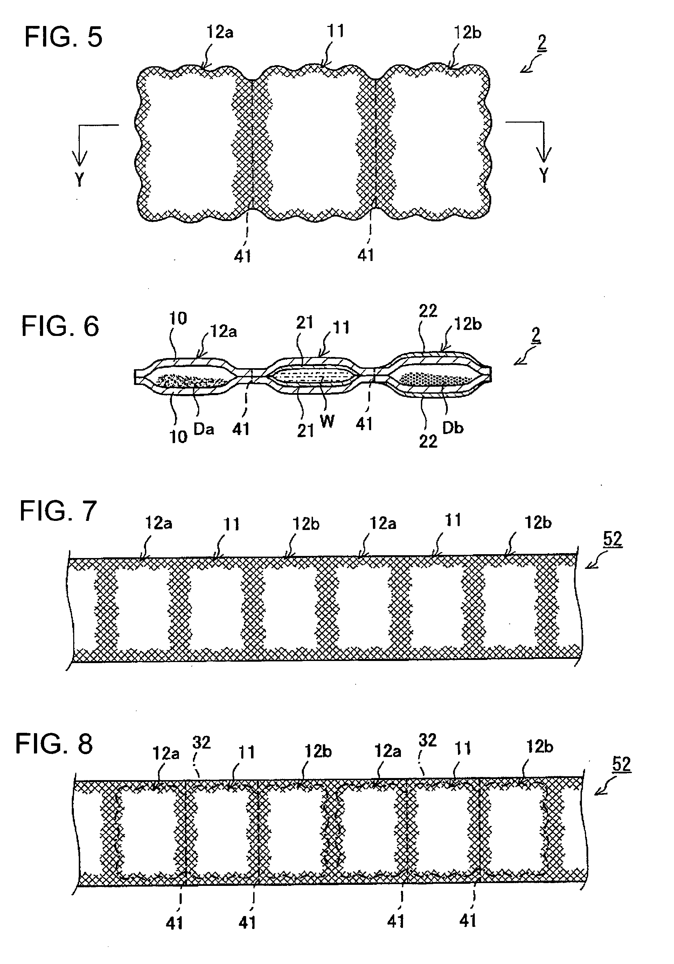

In the above description, the case in which the formulation for oral administration is composed of one liquid storing portion and one drug storing portion has been illustrated, however, a formulation for oral administration 2 which is composed of one liquid storing portion 11 and two drug storing portions 12a, 12b can be formed, as shown in Fig. 5 and Fig. 6. Here, Fig. 5 is a planar view of the formulation for oral administration 2 and Fig. 6 is a cross-sectional view cut along the line Y-Y in Fig. 5. Fig.7 and Fig. 8 are planar views illustrating a method of producing the formulation for oral administration in Fig. 5. Meanwhile, in Fig. 6, the dimension in the direction of the thickness is enlarged for showing a structure apparently. Further, shaded portions in planer views show bonded portions at which edible films are bonded.

As shown in Fig. 5, the formulation for oral administration 2 has a structure in which the drug storing portion 12b is also continuously designed to the formulation for oral administration 1, the drug storing portion 12b is positioned at opposite side to the drug storing portion 12a with respect to the liquid storing portion 11. Namely, both of the two drug storing portions 12a, 12b are adjacent to the liquid storing portion 11.

Also, in the drug storing portion 12b of the formulation for oral administration 2, enteric layers 22 are laminated outside the water-soluble edible films 10. Further, a drug Db is stored in the drug storing portion 12b and a kind of drug component of the drug Db is different from that of the drug Da stored in the drug storing portion 12a.

The formulation for oral administration 2 having such a structure can be produced in the same manner as in the formulation for oral administration 1. However, a step of laminating the enteric layers 22 is required in addition to the above production steps. The step can be performed by partially coating a solution of the enteric material with application, spraying, printing or the like over a portion corresponding to the drug storing portion 12b in a surface opposite to a surface to which the water-insoluble layers 21 are laminated with respect to the water-soluble edible films 10. The other steps can be performed in the same manner as those described above. However, as shown in Fig. 7, a formulation sheet 52 is produced such that the drug storing portion 12a, the liquid storing portion 11 and the drug storing portion 12b are continuously designed repeatedly in this order as a unit. Meanwhile, in the formulation sheet 52, the drug storing portion 12a is adjacent to the right side of the liquid storing portion 11 in a planar view, and the drug storing portion 12b is adjacent to the left side of the liquid storing portion 11 in a planar view.

Then, as shown in Fig. 8, if the formulation for oral administration 2 shown in Fig. 5 and Fig. 6 can be obtained by cutting the formulation sheet 52 along dashed-dotted lines 32 shown in the drawing in the bonded portions. Further, perforated lines 41 can be provided between the drug storing portions 12a and the liquid storing portions 11 and between the liquid storing portions 11 and the drug storing portions 12b in the same manner as that described above.

With the formulation for oral administration 2 having a structure as described above, the enteric layers 22 are provided outside the drug storing portion 12b so that the drug Db can be absorbed through the intestine even though the drug Db itself is water-soluble.

Further, although it is difficult to swallow the drug storing portion 12b outside which the enteric layers 22 are laminated as it is because the drug storing portion 12b doesn't dissolve or collapse in the oral cavity, the drug storing portion 12b can be easily swallowed without separately preparing water or the like since a liquid is supplied from the adjacent liquid storing portion 11.

Further, different kinds of drugs Da, Db respectively stored in the two drug storing portions 12a, 12b can be simultaneously taken with the aqueous liquid W stored in one liquid storing portion 11. In addition, if drugs Da, Db are simultaneously taken, one drug Da can be absorbed through the mucosa in the oral cavity while the other drug Db can be absorbed through the intestine.

Also, since the enteric layers 22 are provided outside the drug storing portion 12b, the drug storing portion can be made thinner than the case where the enteric layers are provided inside the drug storing portion so as to be easily swallowed. That is, in the case where the enteric layers are provided inside the drug storing portion 12b, the enteric layers are required to have enough strength to maintain a state that the layers contain the drug 12b therein when the water-soluble edible films 10 dissolves with saliva. Therefore the enteric layer is required to be thicker. With respect thereto, in the present embodiment, the enteric layers 22 provided outside the drug storing portion 12b can be made thinner since it is sufficient that the layers have the strength to the extent that the layers can provide the water-soluble edible films 10 with an enteric property.

Further, both of the above formulations for oral administration 1, 2 have external shapes formed into a waveform so that a user feels soft in the oral cavity, and doesn't feel discomfort in their oral cavities. Also, a user can take the formulations with a sense of safety since the formulations have no sharp angled portion or linear portion.

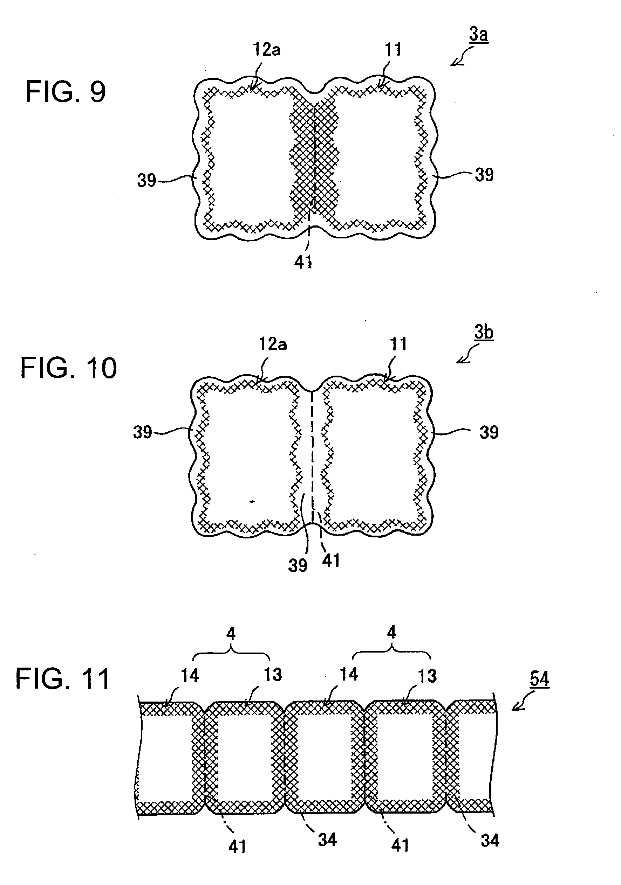

Meanwhile, as shown Fig. 9, in each of the outer circumferences of three sides of the liquid storing portion 11 not adjacent to the drug storing portion 12a and the outer circumferences of three sides of the drug storing portion 12a not adjacent to the liquid storing portion 11, a formulation for oral administration 3a in which not-bonded portions 39 not to be bonded are formed outside the bonded portions can be produced. Therefore, with saliva, the water-soluble edible films 10 easily dissolve at the not-bonded portions 39 and feeling in the oral cavity is softened.

In addition, as in a formulation for oral administration 3b shown in Fig. 10, the not-bonded portion 39 is formed along the outer circumference of the bonded portion of the liquid storing portion 11 and the not-bonded portion 39 is formed along the outer circumference of the bonded portion of the drug storing portion 12a so that the not-bonded portion 39 is interposed at the boundary of the liquid storing portion 11 and the drug storing portion 12a. Therefore, when the formulation for oral administration 3b is taken, the formulation is easily folded at the not-bonded portion 39. The formulation for oral administration 3b doesn't take much space in the oral cavity because the liquid storing portion 11 and the drug storing portion 12a can be taken in a folded and overlapped manner.

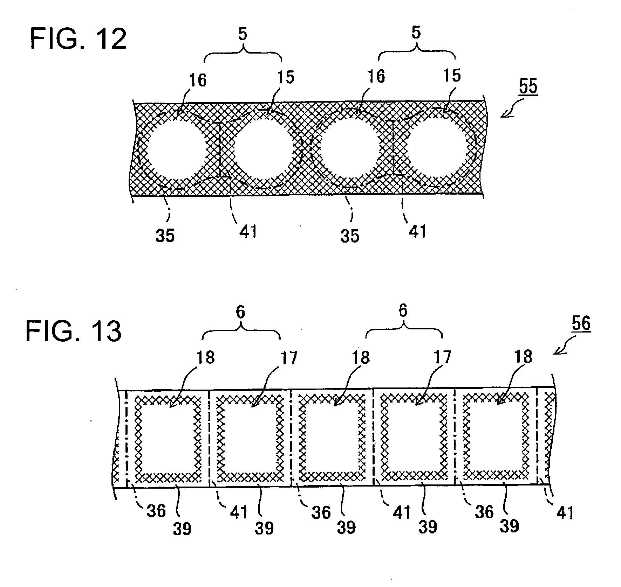

For example, shapes of a formulation for oral administration and a formulation sheet are not limited to the above described ones, as shown in Fig. 11, a formulation sheet 54 can be produced in which substantially quadrangular liquid storing portions 13 and drug storing portions 14 of which angled portions are rounded respectively are continuously designed one after another. In this case, the formulation sheet 54 may be cut along dashed-dotted lines 34 shown in the drawing so that the formulation for oral administration 4 as a unit may be previously cut and separated. Or the dashed-dotted lines 34 shown in the drawing may be used as perforated lines or scores for cutting, and the formulation for oral administration 4 which includes one liquid storing portion 13 and one drug storing portion 14 as a unit may be taken by cutting the formulation by a user. Alternatively, as shown in Fig. 12, a formulation sheet 55 is produced such that inner circumferences of the bonded portions in liquid storing portions 15 and drug storing portions 16 are substantially circular shapes in a planar view, and the sheet is cut along dashed-dotted lines 35 shown in the drawing so that rounded-shape formulations for oral administration 5 can be obtained. Meanwhile, the perforated line 41 for folding can be provided also in the formulation sheet 54 and the formulation sheet 55.

Alternatively, as shown in Fig. 13, substantially quadrangular liquid storing portions 17 and drug storing portions 18 are continuously designed one after another, and a formulation sheet 56 can be produced in which the not-bonded portions 39 are formed outside the bonded portions in the liquid storing portions 17 and the drug storing portions 18. In this case, the formulation sheet 56 may be cut along dashed-dotted lines 36 shown in the drawing and the formulation for oral administration 6 as a unit may be previously cut and separated, or the dashed-dotted lines 36 shown in the drawing may be used as perforated lines or scores for cutting. Also, perforated lines 41 for folding can be provided. Thus, the not-bonded portions 39 are provided at the peripheries of the liquid storing portions 17 and the drug storing portions 18 so that feeling in the oral cavity is softened and, at the same time, feeling in one's hands when taken and physical appearance are also softened. Therefore unlike the above described formulation, if external shape of the formulation for oral administration is not formed into a waveform or rounded shape, the formulations for oral administration 6, which can be easily taken without a sense of resistance, can be made. In addition, unlike the case where external shape of the formulation for oral administration is formed in to a waveform or rounded shape, edges or angled portions of a film are not required to be cut and discarded so that resources can be effectively utilized without waste.

Further, the invention is not limited to the case where the liquid storing portion and the drug storing portion are continuously designed in a line, and they may be designed in two lines or three lines. For example, a formulation for oral administration including one liquid storing portion and one drug storing portion can be obtained by producing a formulation sheet having a structure of two lines where one line formed by only the liquid storing portion and one line formed by only the drug storing portion are lined and cutting the formulation sheet in the direction orthogonal to the direction of lines.

And, in the above description, the case where the enteric layers 22 are laminated outside the water-soluble edible films 10 as substrates to form the liquid storing portion 12b has been described, however, the invention is not limited thereto. For example, a dug storing portion may be formed with enteric edible films as substrates. Thus, when kinds of edible films to be substrates are different between the liquid storing portion and the drug storing portion, the liquid storing portion and the drug storing portion are overlapped on one another at some portion and bonded so that they can be made adjacent to each other. Alternatively, a formulation for oral administration in which the drug storing portion is adjacent to the liquid storing portion can be obtained by flow-casting of a water-soluble film forming agent which forms the liquid storing portion and an enteric film forming agent which forms the drug storing portion in lines.

In addition, in the above description, the formulation for oral administration 2 in which the enteric layers 22 are provided outside the water-soluble edible films 10 in the drug storing portion 12b has been exemplified, a structure in which thin film layers of a metal or metal oxide instead of the enteric layers or gastric-soluble layers are formed may be employed. Such thin film layers are formed by sputtering a metal such as gold, silver and platinum, recognized as food additives or metal oxide such as aluminum oxide, titanium oxide and magnesium oxide, and silicon oxide. Thereby, the drug storing portion is made to be difficult to dissolve or collapse in the oral cavity and resistance to light and resistance to acid can be provide to edible films and drug which form the drug storing portion. Also, contents stored in the liquid storing portion or the drug storing portion can be identified by making kinds of substances constituting the thin film layers different.

Further, characteristic information, symbols or the like can be indicated on a formulation for oral administration by subjecting the edible films to printing using an edible ink, embossing process of an edible metal such as gold or silver or embossing process of edible color foil. Therefore, for example, even when a drug which is difficult to be discriminated by appearance, such as white powder agent, is stored, the drug can be identified by the indication thereby preventing inappropriate uses. Meanwhile, embossing of an edible color foil can be performed by heat-sealing an embossing material provided with a color layer in which an edible dye or the like is mixed with an edible adhesive such as shellac or zein, to an edible film.

## Claims

1. A formulation for oral administration **characterized by** comprising:
a liquid storing portion in which peripheries of overlaid water-soluble edible films are bonded, a closed space is formed inside the films, water-insoluble layers are laminated inside the water-soluble edible films and an aqueous liquid is stored inside the layers, and
a drug storing portion which is adjacent to the liquid storing portion and in which peripheries of overlaid edible films are bonded and a closed space is formed inside the films and a drug is stored inside the space.

2. The formulation for oral administration according to Claim 1, **characterized in that** the edible films constituting the drug storing portion are formed with the same film forming agent as the water-soluble edible films constituting the liquid storing portion, and
the edible films constituting the drug storing portion are laminated with the gastric-soluble or enteric layer.

3. The formulation for oral administration according to Claim 1, **characterized by** being constituted by one liquid storing portion and two drug storing portions.

4. The formulation for oral administration according to Claim 1, **characterized in that** each of the liquid storing portion and drug storing portion includes a not-bonded portion which is not bonded along at least a portion of the outer circumference of the peripheral bonded portion.

## Patentansprüche

1. Formulierung zur oralen Verabreichung, **dadurch gekennzeichnet, dass** sie umfasst:
einen Flüssigkeitsspeicherabschnitt, in welchem Peripherien überlagerter wasserlöslicher essbarer Filme verbunden sind, wobei ein geschlossener Raum innerhalb der Filme gebildet wird, wasserunlösliche Schichten innerhalb der wasserlöslichen essbaren Filme laminiert werden und eine wässrige Flüssigkeit innerhalb der Schichten gespeichert wird, und einen Drogenspeicherabschnitt, welcher an den
Flüssigkeitsspeicherabschnitt angrenzt und in welchem Peripherien überlagerter essbarer Filme verbunden sind und ein geschlossener Raum innerhalb der Filme gebildet wird und eine Droge innerhalb des Raums gelagert wird.

2. Formulierung zur oralen Verabreichung nach Anspruch 1, **dadurch gekennzeichnet, dass** die essbaren Filme, welche den Drogenspeicherabschnitt darstellen, mit demselben Filmbildungsmittel gebildet werden wie die wasserlöslichen essbaren Filme, welche den Flüssigkeitsspeicherabschnitt darstellen, und
die essbaren Filme, welche den Drogenspeicherabschnitt darstellen, mit der im Magen löslichen oder enterischen Schicht laminiert werden.

3. Formulierung zur oralen Verabreichung nach Anspruch 1, **dadurch gekennzeichnet, dass** sie aus einem Flüssigkeitsspeicherabschnitt und zwei Drogenspeicherabschnitten besteht.

4. Formulierung zur oralen Verabreichung nach Anspruch 1, **dadurch gekennzeichnet, dass** sowohl der Flüssigkeitsspeicherabschnitt als auch der Drogenspeicherabschnitt einen nicht-verbundenen Abschnitt beinhaltet, welcher nicht entlang wenigstens eines Abschnitts des äußeren Umfangs des peripheren verbundenen Bereichs verbunden ist.

## Revendications

1. Formulation pour administration orale, **caractérisée en ce qu'**elle comprend:
une portion de stockage de liquide dans laquelle sont liées des périphéries de films comestibles superposés solubles dans l'eau, un espace fermé est formé à l'intérieur des films, des couches insolubles dans l'eau sont stratifiées à l'intérieur des films comestibles solubles dans l'eau et un liquide aqueux est stocké à l'intérieur des couches, et
une portion de stockage de médicament qui est adjacente à la portion de stockage de liquide et dans laquelle sont liées des périphéries de films comestibles superposés solubles dans l'eau et un espace est formé à l'intérieur des films et un médicament est stocké à l'intérieur de l'espace.

2. Formulation pour administration orale selon la revendication 1, **caractérisée en ce que** les films comestibles qui constituent la portion de stockage de médicament sont formés avec le même agent de formation de film que les films comestibles solubles dans l'eau qui constituent la portion de stockage de liquide, et
les films comestibles qui constituent la portion de stockage de médicament sont stratifiés avec la couche soluble en milieu gastrique ou entérique.

3. Formulation pour administration orale selon la revendication 1, **caractérisée en ce qu'**elle est constituée d'une portion de stockage de liquide et de deux portions de stockage de médicament.

4. Formulation pour administration orale selon la revendication 1, **caractérisée en ce que** chacune de la portion de stockage de liquide et de la portion de stockage de médicament inclut une portion non liée qui n'est pas liée le long d'au moins une portion de la circonférence extérieure de la portion périphérique liée.
